# EUROPEAN PATENT SPECIFICATION

(11) **EP 1 898 803 B1**
(45) Date of publication and mention of the grant of the patent: **25.04.2012**
(21) Application number: 06774185.0
(22) Date of filing: 27.06.2006
(51) Int. Cl.: C12Q 1/68, C12P 19/34, C07H 21/04

(54) **MOLECULAR/GENETIC ABERRATIONS IN SURGICAL MARGINS OF RESECTED PANCREATIC CANCER REPRESENTS NEOPLASTIC DISEASE THAT CORRELATES WITH DISEASE OUTCOME**
MOLEKULARE/GENETISCHE ABERRATIONEN IN CHIRURGISCHEN RÄNDERN VON PANKREASKREBSRESEKTIONEN STELLEN EINE NEOPLASTISCHE ERKRANKUNG DAR, DIE MIT DEM RESULTAT KORRELIERT
ABERRATIONS MOLÉCULAIRES/GÉNÉTIQUES DÉTECTÉES DANS LES MARGES CHIRURGICALES D'UN CANCER DU PANCRÉAS RESÉQUÉ REPRÉSENTANT UNE MALADIE NÉOPLASIQUE CORRESPONDANT À L'ÉVOLUTION DE LA MALADIE

(30) Priority: 27.06.2005 US 694383 P
(43) Date of publication of application: 19.03.2008
(73) Proprietor: John Wayne Cancer Institute, Santa Monica, CA 90404 (US)
(72) Inventor: HOON, David, S., B., Los Angeles, CA 90404 (US); KIM, Joseph, Los Angeles, CA 90049 (US)
(74) Representative: Dehmel, Albrecht
(86) International application number: PCT/US2006/025165
(87) International publication number: WO 2007/002746

(56) References cited:
- US-A- 5 910 407
- US-B1- 6 300 081
- NIEDERGETHMANN MARCO ET AL: "Prognostic implications of routine, immunohistochemical, and molecular staging in resectable pancreatic adenocarcinoma" AMERICAN JOURNAL OF SURGICAL PATHOLOGY, RAVEN PRESS, NEW YORK, NY, US, vol. 26, no. 12, 1 December 2002 (2002-12-01), pages 1578-1587, XP009125016 ISSN: 0147-5185
- MASASYESVA BRETT G ET AL: "Molecular margin analysis predicts local recurrence after sublobar resection of lung cancer." INTERNATIONAL JOURNAL OF CANCER. JOURNAL INTERNATIONAL DU CANCER 1 MAR 2005, vol. 113, no. 6, 1 March 2005 (2005-03-01), pages 1022-1025, XP002553339 ISSN: 0020-7136
- YAMADA SHINOBU ET AL: "A novel high-specificity approach for colorectal neoplasia: Detection of K-ras2 oncogene mutation in normal mucosa." INTERNATIONAL JOURNAL OF CANCER. JOURNAL INTERNATIONAL DU CANCER 1 MAR 2005, vol. 113, no. 6, 1 March 2005 (2005-03-01), pages 1015-1021, XP002553340 ISSN: 0020-7136
- IZAWA T ET AL: "Clonality and field cancerization in intraductal papillary-mucinous tumors of the pancreas." CANCER 1 OCT 2001, vol. 92, no. 7, 1 October 2001 (2001-10-01), pages 1807-1817, XP002553341 ISSN: 0008-543X
- NIEDERGETHMANN M ET AL: "[Detection of micrometastases after curative resection for ductal adenocarcinoma of the pancreas]" ZENTRALBLATT FÜR CHIRURGIE NOV 2001, vol. 126, no. 11, November 2001 (2001-11), pages 917-921, XP009125009 ISSN: 0044-409X
- LUTTGES J ET AL: "The K-ras mutation pattern in pancreatic ductal adenocarcinoma usually is identical to that in associated normal, hyperplastic, and metaplastic ductal epithelium" CANCER, AMERICAN CANCER SOCIETY, PHILADELPHIA, PA, US, vol. 85, no. 8, 15 April 1999 (1999-04-15), pages 1703-1710, XP008111917 ISSN: 0008-543X
- LÜTTGES J ET AL: "The retroperitoneal resection margin and vessel involvement are important factors determining survival after pancreaticoduodenectomy for ductal adenocarcinoma of the head of the pancreas." VIRCHOWS ARCHIV : AN INTERNATIONAL JOURNAL OF PATHOLOGY SEP 1998, vol. 433, no. 3, September 1998 (1998-09), pages 237-242, XP002553342 ISSN: 0945-6317
- MASASYESVA, B.G.: 'Molecular margin analysis predicts local recurrence after sublobar resection lung cancer.' INT.J.CANCER vol. 113, 01 March 2005, pages 1022 - 1025, XP002553339

## Description

### I. Field of the Invention

The present invention relates to the fields of oncology, genetics and molecular biology. More particular the invention relates to the identification, in the margins of resected tumors, genetic defects in cells. Such defects in this gene are associated with the development of cancer, particularly using K-ras mutations in the identification of pancreatic, colorectal, and lung cancer.

### II. Related Art

Pancreatic ductal adenocarcinoma (PDAC) has amongst the worst 5-year survival rates of any cancer (Jermal *et al.,* 2004). A predominant factor for such abysmal outcomes is the insidious onset of disease and the concomitant difficulties in early diagnosis. Similar to many other cancers, cure for PDAC can be obtained when metastases have not occurred and surgery is feasible and safe. Even under optimal conditions, however, the outcomes of curative surgery are often poor with median survival only 6 to 12 months longer than unresectable tumors (Nitecki *et al.,* 1995; Warshaw and Fernandez-del Castillo, 1992; Yeo *et al.,* 1997).

The locoregional failures that are often the endpoints of curative resection for PDAC (Evans *et al.,* 2001; Westerdahl *et al.,* 1993; Griffin *et al.,* 1990; Willett *et al.,* 1993; Greene *et al.,* 2002). may be indicative of the inadequate removal of all neoplastic or precursor tissues despite histopathology negative surgical margins. These failures may be representative of field cancerization or occult metastatic spread of neoplastic cells. The "field cancerization," a concept first introduced by Slaughter *et al.* (1953) who proposed that abnormal tissues surrounding oral squamous cell carcinoma was the source of subsequent primary tumors or locally recurrent cancers. Slaughter identified abnormal tissues by standard histopathologic techniques available in the 1950s. Advances in molecular techniques have led to the identification of genetic and epigenetic defects in malignant and benign appearing cells alike (Brennan *et al.,* 1995; Garcia *et al.,* 1999; Tian *et al.,* 1998; Califano *et al.,* 1996;. Wernert *et al.,* 2001; Monifar *et al.,* 2000; Deng *et al.,* 1996; Ito *et al.,* 2005; Guo *et al.,* 2004; Nathan *et al.,* 2002; Nathan *et al.,* 1999; Temam *et al.,* 2004; tabor *et al.,* 2004; Jassem *et al.,* 2004; Masasyesva *et al.,* 2005; Izawa *et al.,* 2001; Nathan *et al.,* 1997; Braakhuis *et al.,* 2003).

Molecular and genetic data support a model that details the development of a field in which genetically altered cells play a central role in cancer recurrences. Initially, genetic transformation may focally occur in a group of cells forming a lesion of altered cells (Braakhuis *et al.,* 2003). The expansion of this local field into a wider area underscores the critical steps in epithelial carcinogenesis (Braakhuis *et al.,* 2003). Additional molecular/genetic alterations likely occur in a Vogel-gram-like progression (Cubilla and Fitzgerald, 1975) resulting in striking alterations in cell physiology that is representative of malignancy (Hanahan and Weinberg, 2000). By virtue of its growth advantage, a proliferating field gradually displaces the normal mucosa. This field cancerization concept is particularly relevant for PDAC, which has mounting evidence supporting a progressive accumulation of genetic defects, gene silencing, and proto-oncogene activation that is histologically represented by the sequential transformation of benign pancreas to pancreas intraepithelial neoplasia (PanIN) to frank malignancy (Cubilla and Fitzgerald, 1975; Hruban *et al.,* 2000a; Hruban *et al.,* 2000b; Klimstra and Longnecker, 1994).

The genetic defects that comprise the genotypic features of PDAC may coexist in benign appearing cells adjacent to the primary tumor (Deng *et al.,* 1996) and cannot be assessed intraoperatively by conventional histopathologic techniques. The quintessential implication is that cancerization fields or occult tumor cells may remain after surgery of the primary tumor and may subsequently develop into locoregionally recurrent cancers. This assertion has poignant clinical relevance for PDAC because standard "curative" surgery has resulted in limited survival and obtaining additional surgical margins of resection may carry significant morbidities. Thus, the ability to detect tumor cells would greatly aid clinicians in the assessment and treatment of progressive cancer.

### SUMMARY OF THE INVENTION

Thus, in accordance with the present invention, there is provided methods of assessing surgical margin adequacy in a patient undergoing surgical resection of a tumor. By assessing the adequacy of the surgical margin, one may predict the clinical outcome of a cancer patient undergoing surgical resection. The clinical outcome may be, for example, recurrence of the cancer, progression of the cancer, and/or survival.

In one embodiment, the present invention provides a method of predicting survival in a cancer patient undergoing surgical resection comprising (a) obtaining a tissue sample from the surgical margin of the resected tumor and (b) assessing a nucleic acid or protein in cells of said tissue sample for the occurrence of a mutation in an oncogene. The oncogene may be K-ras. The cancer from which the patient suffers may be, pancreatic cancer.

Assessing may comprise immunologic detection of a protein, or detection of a nucleic acid (e.g., an RNA or a DNA). Detection may further comprise nucleic acid amplification, sequencing, primer extension, Northern or Southern blotting, and/or restriction endonuclease treatment.

If a patient is determined to be at risk for cancer recurrence or progression, further treatment or more aggressive treatment for the cancer may be administered. In addition, more extensive surgical resection may be performed where a mutation in an oncogene is detected in a surgical margin. Likewise, a patient identified a being at less risk for cancer recurrence or progression may not require additional anti-cancer therapy beyond surgical resection or may require a less aggressive treatment, thereby reducing unnecessary exposure to anti-cancer treatment. Moreover, the methods of the present invention may comprise modifying a patient's existing anti-cancer treatment based on an analysis of one or more of the methods herein described.

In another embodiment, there is provided a method for predicting cancer progression in a cancer patient undergoing surgical resection comprising (a) obtaining a tissue sample from the surgical margin of the resected tumor and (b) assessing a nucleic acid or protein in cells of said tissue sample for the occurrence of a mutation in K-ras. In particular embodiments, the cancer may be pancreatic cancer and the nucleic acid or protein in cells of the tissue sample are assessed for the occurrence of a mutation in K-ras. Also disclosed are methods wherein the cancer may be melanoma, colon cancer, or thyroid cancer, and the nucleic acid or protein in cells of the tissue sample are assessed for the occurrence of a mutation in B-raf.

There is provided a method predicting recurrence of cancer in a cancer patient undergoing surgical resection comprising: (a) obtaining a tissue sample from the surgical margin of the resected tumor; and (b) assessing a nucleic acid or protein in cells of said tissue sample for the occurrence of a mutation in an oncogene. In particular, the cancer may be pancreatic cancer, and the mutation of interest may be in the K-ras gene.

The present invention provides a method of predicting survival in a cancer patient undergoing surgical resection comprising: (a) assessing a nucleic acid or protein in cells of a tissue sample obtained from the surgical margin of a resected tumor for the occurrence of a mutation in K-ras, wherein the occurrence of the mutation in the tissue sample is predictive of decreased survival; and (b) predicting the survival in the cancer patient.

In another embodiment, the present invention provides a method of predicting cancer progression in a cancer patient undergoing surgical resection comprising: (a) assessing a nucleic acid or protein in cells of a tissue sample obtained from the surgical margin of a resected tumor for the occurrence of a mutation in K-ras, wherein the occurrence of the mutation in the tissue sample is predictive of cancer progression; and (b) predicting the cancer progression in the cancer patient.

In yet another embodiment, the present invention provides a method of predicting recurrence of cancer in a cancer patient undergoing surgical resection comprising (a) assessing a nucleic acid or protein in cells of a tissue sample obtained from the surgical margin of a resected tumor for the occurrence of a mutation in K-ras, wherein the occurrence of the mutation in the tissue sample is predictive of predicting recurrence of cancer; and (b) predicting the recurrence of cancer in the cancer patient.

Assessing the occurrence of a mutation in K-ras may be useful in predicting the clinical outcome (e.g., recurrence, progression, survival) in a cancer patient suffering from a cancer associated with a mutation in K-ras. In certain aspects of the invention the cancer is an epithelial cancer. The cancer may be, for example, pancreatic cancer.

Also disclosed is a method of detecting field cancerization in a cancer patient undergoing surgical resection comprising (a) assessing a nucleic acid or protein in cells of a tissue sample obtained from the surgical margin of a resected tumor for the occurrence of a mutation in K-ras, wherein the occurrence of the mutation in the tissue sample indicates field cancerization; and (b) detecting field cancerization in the cancer patient.

Also disclosed is a method of predicting survival in a cancer patient undergoing surgical resection comprising: (a) assessing a nucleic acid or protein in cells of a tissue sample obtained from the surgical margin of a resected tumor for the occurrence of a mutation in B-raf, wherein the occurrence of the mutation in the tissue sample is predictive of decreased survival; and (b) predicting the survival in the cancer patient.

Also disclosed is a method of predicting cancer progression in a cancer patient undergoing surgical resection comprising: (a) assessing a nucleic acid or protein in cells of a tissue sample obtained from the surgical margin of a resected tumor for the occurrence of a mutation in B-raf, wherein the occurrence of the mutation in the tissue sample is predictive of cancer progression; and (b) predicting the cancer progression in the cancer patient.

Also disclosed is a method of predicting recurrence of cancer in a cancer patient undergoing surgical resection comprising (a) assessing a nucleic acid or protein in cells of a tissue sample obtained from the surgical margin of a resected tumor for the occurrence of a mutation in B-raf, wherein the occurrence of the mutation in the tissue sample is predictive of predicting recurrence of cancer; and (b) predicting the recurrence of cancer in the cancer patient.

Assessing the occurrence of a mutation in B-raf may be useful in predicting the clinical outcome (e.g., recurrence, progression, survival) in a cancer patient suffering from a cancer associated with a mutation in B-raf. The cancer may be for example, colon cancer, melanom, or thyroid cancer. Also disclosed are methods that involve assessing the occurrence of mutations in both K-ras and B-raf.

Also disclosed is a method of detecting field cancerization in a cancer patient undergoing surgical resection comprising (a) assessing a nucleic acid or protein in cells of a tissue sample obtained from the surgical margin of a resected tumor for the occurrence of a mutation in B-raf, wherein the occurrence of the mutation in the tissue sample indicates field cancerization; and (b) detecting field cancerization in the cancer patient. In certain aspects of the invention, the methods involve assessing the occurrence of mutations in both K-ras and B-raf.

It is contemplated that any method or composition described herein can be implemented with respect to any other method or composition described herein. The use of the word "a" or "an" when used in conjunction with the term "comprising" in the claims and/or the specification may mean "one," but it is also consistent with the meaning of "one or more," "at least one," and "one or more than one." "About" means plus or minus 5% of the stated value.

The embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein:
**FIGs. 1A-1B** - Representative sequencing of PDAC tissues to validate PNA PCR results. **(****FIG. 1A****)** Pancreatic cancer specimen with mutant Kras (GGT → GAT). **(****FIG. 1B****)** Pancreatic cancer margin specimen with mutant Kras (GGT → GTT).
**FIG. 2** - Kaplan-Meier curves comparing overall survival between patients with KRAS mutation positive and negative surgical margins.

### DETAILED DESCRIPTION OF THE PREFERRED EMBODIMENTS

The inventor hypothesized that histologically benign tissues in the surgical margins of PDAC harbor the *KRAS* mutation and detection thereof correlates with clinical outcomes. The studies described herein provide evidence that field cancerization or occult metastasis occurs in PDAC and that measures should be implemented to better detect such defects in the margins of resection at the time of surgery.

For this report, the inventor evaluated the *KRAS* DNA mutation. This is the first report demonstrating the pathological utility of *KRAS* mutation in the margins to clinical and pathological utility. Recently, Hingorani *et al.* directed endogenous expression of *KRAS* in a mouse model and recapitulated the entire progression of PDAC (Hingorani *et al.,* 2003), demonstrating the key role of KRAS-activating mutations in pancreatic cancer pathogenesis. KRAS mutations can also be used to identify field cancerization in other cancers, such as lung cancer and colorectal cancer.

### I. Diagnosing Metastasis or Cancerization In the Organ Site

The present invention is drawn to methods of examining cells at the margin of a tumor resection to detect field cancerization or occult metastasis. Though the exemplified methods utilize PCR in a genetic analysis, many other methods may be utilized, as discussed below.

### A. Genetic Diagnosis

Genetic diagnosis looks at abberrations in the genes of a cell - in coding regions, introns, or regulatory regions such as promoters, enhancers and terminators. The mutations may be point mutants, rearrangements, duplications and deletion (including truncations). Obviously, this sort of assay has importance in the diagnosis of related solid tumor cancers. In particular, the present disclosure relates to the diagnosis of pancreatic cancers, as well as colorectal and lung cancers associated with K-ras, and to the diagnosis of melanoma, thyroid and colorectal cancers associated with B-raf.

The nucleic acid used is isolated from cells contained in the biological sample obtained from the tumor margin according to standard methodologies (Sambrook *et al.,* 1989). The nucleic acid may be, for example, genomic DNA. Normally, the nucleic acid is amplified.

Depending on the format, the specific nucleic acid of interest is identified in the sample directly using amplification or with a second, known nucleic acid following amplification. Next, the identified product is detected. In certain applications, the detection may be performed by visual means (*e.g*., ethidium bromide staining of a gel). Alternatively, the detection may involve indirect identification of the product via chemiluminescence, radioactive scintigraphy of radiolabel or fluorescent label or even via a system using electrical or thermal impulse signals (Affymax Technology; Bellus, 1994). In certain embodiments, the nucleic acid of interest is identified using quatitative realtime PCR, such as with TaqMan® probes (Applied Biosystems).

Various types of defects have been identified by the present inventors. Thus, "alterations" should be read as including deletions, insertions, point mutations and duplications. Point mutations result in stop codons, frameshift mutations or amino acid substitutions. Somatic mutations are those occurring in non-germline tissues. Germ-line tissue can occur in any tissue and are inherited. Mutations in and outside the coding region also may affect the amount of protein produced, both by altering the transcription of the gene or in destabilizing or otherwise altering the processing of either the transcript (mRNA) or protein.

A cell takes a genetic step toward oncogenic transformation when one allele of a tumor suppressor gene is inactivated due to inheritance of a germline lesion or acquisition of a somatic mutation. The inactivation of the other allele of the gene usually involves a somatic micromutation or chromosomal allelic deletion that results in loss of heterozygosity (LOH). Alternatively, both copies of a gene may be lost by homozygous deletion.

### (i) Primers and Probes

The term primer, as defined herein, is meant to encompass any nucleic acid that is capable of priming the synthesis of a nascent nucleic acid in a template-dependent process. Typically, primers are oligonucleotides from ten to twenty base pairs in length, but longer sequences can be employed. Primers may be provided in double-stranded or single-stranded form, although the single-stranded form is preferred. Probes are defined differently, although they may act as primers. Probes, while perhaps capable of priming, are designed to binding to the target nucleic acid and need not be used in an amplification process.

In preferred embodiments, the probes or primers are labeled with radioactive species (³²P, ¹⁴C, ³⁵S, ³H, or other label), with a fluorophore (rhodamine, fluorescein) or a chemillumiscent (luciferase).

One particular main assay for *KRAS* mutations is DNA-based amplification and/or sequencing. mRNA may be used, normally after RT-PCT. The DNA assay may also be PCR-based, using PNA (peptide nucleic acid) in the assay to clamp discriminating wild-type *KRAS* to mutated KRAS. The assay is quantitative by real time PCR™. The assay for occult colorectal cancer cells in draining lymph nodes was first reported by Taback *et al.* (2004).

### (ii) Template Dependent Amplification Methods

A number of template dependent processes are available to amplify the marker sequences present in a given template sample. One of the best known amplification methods is the polymerase chain reaction (referred to as PCR^{™}) which is described in detail in U.S. Patent Nos. 4,683,195, 4,683,202 and 4,800,159, and in Innis et al., 1990

Briefly, in PCR™, two primer sequences are prepared that are complementary to regions on opposite complementary strands of the marker sequence. An excess of deoxynucleoside triphosphates are added to a reaction mixture along with a DNA polymerase, *e.g., Taq* polymerase. If the marker sequence is present in a sample, the primers will bind to the marker and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated.

A reverse transcriptase PCR™ amplification procedure may be performed in order to quantify the amount of mRNA amplified. Methods of reverse transcribing RNA into cDNA are well known and described in Sambrook *et al.,* 1989. Alternative methods for reverse transcription utilize thermostable, RNA-dependent DNA polymerases. These methods are described in WO 90/07641 filed December 21, 1990. Polymerase chain reaction methodologies are well known in the art.

Another method for amplification is the ligase chain reaction ("LCR"), disclosed in EPO No. 320 308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR™, bound ligated units dissociate from the target and then serve as "target sequences" for ligation of excess probe pairs. U.S. Patent 4,883,750 describes a method similar to LCR for binding probe pairs to a target sequence.

Qbeta Replicase, may also be used as still another amplification method in the present invention. In this method, a replicative sequence of RNA that has a region complementary to that of a target is added to a sample in the presence of an RNA polymerase. The polymerase will copy the replicative sequence that can then be detected.

An isothermal amplification method, in which restriction endonucleases and ligases are used to achieve the amplification of target molecules that contain nucleotide 5'-[alpha-thio]-triphosphates in one strand of a restriction site may also be useful in the amplification of nucleic acids in the present invention, Walker *et al.,* (1992).

Strand Displacement Amplification (SDA) is another method of carrying out isothermal amplification of nucleic acids which involves multiple rounds of strand displacement and synthesis, *i.e*., nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA. Target specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

Still another amplification methods described in GB Application No. 2 202 328, may be used in accordance with the present invention. In the former application, "modified" primers are used in a PCR™-like, template- and enzyme-dependent synthesis. The primers may be modified by labeling with a capture moiety (*e.g*., biotin) and/or a detector moiety (*e.g*., enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS), including nucleic acid sequence based amplification (NASBA) and 3SR (Kwoh *et al.,* 1989; Gingeras et al., PCT Application WO 88/10315 ). In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has target specific sequences. Following polymerization, DNA/RNA hybrids are digested with RNase H while double stranded DNA molecules are heat denatured again. In either case the single stranded DNA is made fully double-stranded by addition of second target specific primer, followed by polymerization. The double-stranded DNA molecules are then multiply transcribed by an RNA polymerase such as T7 or SP6. In an isothermal cyclic reaction, the RNA's are reverse transcribed into single-stranded DNA, which is then converted to double stranded DNA, and then transcribed once again with an RNA polymerase such as T7 or SP6. The resulting products, whether truncated or complete, indicate target specific sequences.

Davey et al., EPO No. 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a template for a second primer, which also includes the sequences of an RNA polymerase promoter (exemplified by T7 RNA polymerase) 5' to its homology to the template. This primer is then extended by DNA polymerase (exemplified by the large "Klenow" fragment of *E*. *coli* DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller et al., PCT Application WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic, i.e., new templates are not produced from the resultant RNA transcripts. Other amplification methods include "RACE" and "one-sided PCR™" (Frohman, 1990; Ohara *et al.,* 1989.

Methods based on ligation of two (or more) oligonucleotides in the presence of nucleic acid having the sequence of the resulting "di-oligonucleotide", thereby amplifying the di-oligonucleotide, may also be used in the amplification step of the present invention (Wu *et al.,* 1989).

### (iii) Southern/Northern Blotting

Blotting techniques are well known to those of skill in the art. Southern blotting involves the use of DNA as a target, whereas Northern blotting involves the use of RNA as a target. Each provide different types of information, although cDNA blotting is analogous, in many aspects, to blotting or RNA species.

Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter.

Subsequently, the blotted target is incubated with a probe (usually labeled) under conditions that promote denaturation and rehybridization. Because the probe is designed to base pair with the target, the probe will binding a portion of the target sequence under renaturing conditions. Unbound probe is then removed, and detection is accomplished as described above.

### (iv) Separation Methods

It normally is desirable, at one stage or another, to separate the amplification product from the template and the excess primer for the purpose of determining whether specific amplification has occurred. In one embodiment, amplification products are separated by agarose, agarose-acrylamide or polyacrylamide gel electrophoresis using standard methods. See Sambrook *et al.,* 1989. Alternatively, capillary array electrophoresis or realtime PCR may be used.

### (v) Detection Methods

Products may be visualized in order to confirm amplification of the marker sequences. One typical visualization method involves staining of a gel with ethidium bromide and visualization under UV light. Alternatively, if the amplification products are integrally labeled with radio- or fluorometrically-labeled nucleotides, the amplification products can then be exposed to x-ray film or visualized under the appropriate stimulating spectra, following separation.

In one embodiment, visualization is achieved indirectly. Following separation of amplification products, a labeled nucleic acid probe is brought into contact with the amplified marker sequence. The probe preferably is conjugated to a chromophore but may be radiolabeled. In another embodiment, the probe is conjugated to a binding partner, such as an antibody or biotin, and the other member of the binding pair carries a detectable moiety.

In one embodiment, detection is by a labeled probe. The techniques involved are well known to those of skill in the art and can be found in many standard books on molecular protocols. See Sambrook *et al.* (1989). For example, chromophore or radiolabel probes or primers identify the target during or following amplification.

One example of the foregoing is described in U.S. Patent No. 5,279,721, which discloses an apparatus and method for the automated electrophoresis and transfer of nucleic acids. The apparatus permits electrophoresis and blotting without external manipulation of the gel and is suited to carrying out methods according to the present invention.

In addition, the amplification products described above may be subjected to sequence analysis to identify specific kinds of variations using standard sequence analysis techniques. Within certain methods, exhaustive analysis of genes is carried out by sequence analysis using primer sets designed for optimal sequencing (Pignon *et al.,* 1994). The present invention provides methods by which any or all of these types of analyses may be used. Using the sequences disclosed herein, oligonucleotide primers may be designed to permit the amplification of sequences throughout the target gene that may then be analyzed by direct sequencing.

### (vi) Kit Components

All the essential materials and reagents required for detecting and sequencing targets thereof may be assembled together in a kit. This generally will comprise preselected primers and probes. In certain embodiments, a kit of the present invention may contain primers or probes preselected for the identification of one or more mutations in the *KRAS* gene. Also included may be enzymes suitable for amplifying nucleic acids including various polymerases (RT, Taq, Sequenase^{™} etc.), deoxynucleotides and buffers to provide the necessary reaction mixture for amplification. Such kits also generally will comprise, in suitable means, distinct containers for each individual reagent and enzyme as well as for each primer or probe.

### (vii) Design and Theoretical Considerations for Relative Quantitative RT-PCR™

In PCR™; the number of molecules of the amplified target DNA increase by a factor approaching two with every cycle of the reaction until some reagent becomes limiting. Thereafter, the rate of amplification becomes increasingly diminished until there is no increase in the amplified target between cycles. If a graph is plotted in which the cycle number is on the X axis and the log of the concentration of the amplified target DNA is on the Y axis, a curved line of characteristic shape is formed by connecting the plotted points. Beginning with the first cycle, the slope of the line is positive and constant. This is said to be the linear portion of the curve. After a reagent becomes limiting, the slope of the line begins to decrease and eventually becomes zero. At this point the concentration of the amplified target DNA becomes asymptotic to some fixed value. This is said to be the plateau portion of the curve.

The concentration of the target DNA in the linear portion of the PCR™ amplification is directly proportional to the starting concentration of the target before the reaction began. By determining the concentration of the amplified products of the target DNA in PCR™ reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundances of the specific mRNA from which the target sequence was derived can be determined for the respective tissues or cells. This direct proportionality between the concentration of the PCR™ products and the relative mRNA abundances is only true in the linear range of the PCR™ reaction.

The final concentration of the target DNA in the plateau portion of the curve is determined by the availability of reagents in the reaction mix and is independent of the original concentration of target DNA. Therefore, the first condition that must be met before the relative abundances of a mRNA species can be determined by RT-PCR™ for a collection of RNA populations is that the concentrations of the amplified PCR™ products must be sampled when the PCR™ reactions are in the linear portion of their curves.

The second condition that must be met for an RT-PCR™ experiment to successfully determine the relative abundances of a particular mRNA species is that relative concentrations of the amplifiable cDNAs must be normalized to some independent standard. The goal of an RT-PCR™ experiment is to determine the abundance of a particular mRNA species relative to the average abundance of all mRNA species in the sample. In the experiments described below, mRNAs for B-actin, asparagine synthetase and lipocortin II were used as external and internal standards to which the relative abundance of other mRNAs are compared.

Most protocols for competitive PCR™ utilize internal PCR™ standards that are approximately as abundant as the target. These strategies are effective if the products of the PCR™ amplifications are sampled during their linear phases. If the products are sampled when the reactions are approaching the plateau phase, then the less abundant product becomes relatively over represented. Comparisons of relative abundances made for many different RNA samples, such as is the case when examining RNA samples for differential expression, become distorted in such a way as to make differences in relative abundances of RNAs appear less than they actually are. This is not a significant problem if the internal standard is much more abundant than the target. If the internal standard is more abundant than the target, then direct linear comparisons can be made between RNA samples.

The above discussion describes theoretical considerations for an RT-PCR™ assay for clinically derived materials. The problems inherent in clinical samples are that they are of variable quantity (making normalization problematic), and that they are of variable quality (necessitating the co-amplification of a reliable internal control, preferably of larger size than the target). Both of these problems are overcome if the RT-PCR™ is performed as a relative quantitative RT-PCR™ with an internal standard in which the internal standard is an amplifiable cDNA fragment that is larger than the target cDNA fragment and in which the abundance of the mRNA encoding the internal standard is roughly 5-100 fold higher than the mRNA encoding the target. This assay measures relative abundance, not absolute abundance of the respective mRNA species.

Other studies may be performed using a more conventional relative quantitative RT-PCR™ assay with an external standard protocol. These assays sample the PCR™ products in the linear portion of their amplification curves. The number of PCR™ cycles that are optimal for sampling must be empirically determined for each target cDNA fragment. In addition, the reverse transcriptase products of each RNA population isolated from the various tissue samples must be carefully normalized for equal concentrations of amplifiable cDNAs. This consideration is very important since the assay measures absolute mRNA abundance. Absolute mRNA abundance can be used as a measure of differential gene expression only in normalized samples. While empirical determination of the linear range of the amplification curve and normalization of cDNA preparations are tedious and time consuming processes, the resulting RT-PCR™ assays can be superior to those derived from the relative quantitative RT-PCR™ assay with an internal standard.

One reason for this advantage is that without the internal standard/competitor, all of the reagents can be converted into a single PCR™ product in the linear range of the amplification curve, thus increasing the sensitivity of the assay. Another reason is that with only one PCR™ product, display of the product on an electrophoretic gel or another display method becomes less complex, has less background and is easier to interpret.

### (viii) Chip Technologies

Specifically contemplated by the present inventors are chip-based DNA technologies such as those described by Hacia *et al.* (1996) and Shoemaker *et al.* (1996). Briefly, these techniques involve quantitative methods for analyzing large numbers of genes rapidly and accurately. By tagging genes with oligonucleotides or using fixed probe arrays, one can employ chip technology to segregate target molecules as high density arrays and screen these molecules on the basis of hybridization. See also Pease *et al.* (1994); Fodor *et al.* (1991).

### B. Immunodiagnosis

Antibodies of the present invention can be used in characterizing the protein content of healthy and diseased tissues at the margin of a resected tumor, through techniques such as ELISAs and Western blotting. This may provide a screen for the presence or absence of malignancy or as a predictor of future cancer. In another aspect, the present invention contemplates use of an antibody that is immunoreactive with a target molecule of the present invention, or any portion thereof. An antibody can be a polyclonal or a monoclonal antibody. In a preferred embodiment, an antibody is a monoclonal antibody. Means for preparing and characterizing antibodies are well known in the art (see, *e.g*., Harlow and Lane, 1988).

The use of antibodies of the present invention, in an ELISA assay is contemplated. For example, antibodies are immobilized onto a selected surface, preferably a surface exhibiting a protein affinity such as the wells of a polystyrene microtiter plate. After washing to remove incompletely adsorbed material, it is desirable to bind or coat the assay plate wells with a non-specific protein that is known to be antigenically neutral with regard to the test antisera such as bovine serum albumin (BSA), casein or solutions of powdered milk. This allows for blocking of non-specific adsorption sites on the immobilizing surface and thus reduces the background caused by non-specific binding of antigen onto the surface.

After binding of antibody to the well, coating with a non-reactive material to reduce background, and washing to remove unbound material, the immobilizing surface is contacted with the sample to be tested in a manner conducive to immune complex (antigen/antibody) formation.

Following formation of specific immunocomplexes between the test sample and the bound antibody, and subsequent washing, the occurrence and even amount of immunocomplex formation may be determined by subjecting same to a second antibody having specificity for the target that differs the first antibody. Appropriate conditions preferably include diluting the sample with diluents such as BSA, bovine gamma globulin (BGG) and phosphate buffered saline (PBS)/Tween^{®}. These added agents also tend to assist in the reduction of nonspecific background. The layered antisera is then allowed to incubate for from about 2 to about 4 hr, at temperatures preferably on the order of about 25° to about 27°C. Following incubation, the antisera-contacted surface is washed so as to remove non-immunocomplexed material. A preferred washing procedure includes washing with a solution such as PBS/Tween^{®}, or borate buffer.

To provide a detecting means, the second antibody will preferably have an associated enzyme that will generate a color development upon incubating with an appropriate chromogenic substrate. Thus, for example, one will desire to contact and incubate the second antibody-bound surface with a urease or peroxidase-conjugated anti-human IgG for a period of time and under conditions which favor the development of immunocomplex formation (*e.g*., incubation for 2 hr at room temperature in a PBS-containing solution such as PBS/Tween^{®}).

After incubation with the second enzyme-tagged antibody, and subsequent to washing to remove unbound material, the amount of label is quantified by incubation with a chromogenic substrate such as urea and bromocresol purple or 2,2'-azino-di-(3-ethyl-benzthiazoline)-6-sulfonic acid (ABTS) and H₂O₂, in the case of peroxidase as the enzyme label. Quantitation is then achieved by measuring the degree of color generation, *e.g*., using a visible spectrum spectrophotometer.

The preceding format may be altered by first binding the sample to the assay plate. Then, primary antibody is incubated with the assay plate, followed by detecting of bound primary antibody using a labeled second antibody with specificity for the primary antibody.

The antibody compositions of the present invention will find great use in immunoblot or Western blot analysis. The antibodies may be used as high-affinity primary reagents for the identification of proteins immobilized onto a solid support matrix, such as nitrocellulose, nylon or combinations thereof. In conjunction with immunoprecipitation, followed by gel electrophoresis, these may be used as a single step reagent for use in detecting antigens against which secondary reagents used in the detection of the antigen cause an adverse background. Immunologically-based detection methods for use in conjunction with Western blotting include enzymatically-, radiolabel-, or fluorescently-tagged secondary antibodies against the toxin moiety are considered to be of particular use in this regard.

Another assay is the Mutector^{™} assay from Trimgen. This assay and commerically available kit emplys a technique known as Shift Terminated Assay, which is a specifically designed primer extension method. This approach permits detection of any type of mutations such as SNP, deletion and translocations. It utilizes three steps: (a) sequence specific hybridization, (b) sequence dependent primer extension, and (c) sequence dependent chain termination. PCR is performed prior to treatment to provide sufficient template. Also contemplated is the method of Feinberg & Vogelstein (1983).

### II. Nucleic Acids

In accordance with the present invention, one may wish to purifiy, identify and/or amplify nucleic acids encoding a target gene of interest (tumor suppressor, oncongene, proapoptotis gene, cell cycle regulator), such as K-ras or B-raf. The nucleic acid may be derived from genomic DNA, *i.e*., cloned directly from the genome of a particular organism. In preferred embodiments, however, the nucleic acid would comprise complementary DNA (cDNA). Also contemplated is a cDNA plus a natural intron or an intron derived from another gene; such engineered molecules are sometime referred to as "mini-genes." At a minimum, these and other nucleic acids of the present invention may be used as molecular weight standards in, for example, gel electrophoresis.

Naturally, the present invention also encompasses DNA segments that are complementary, or essentially complementary, to target sequences. Nucleic acid sequences that are "complementary" are those that are capable of base-pairing according to the standard Watson-Crick complementary rules. As used herein, the term "complementary sequences" means nucleic acid sequences that are substantially complementary, as may be assessed by the same nucleotide comparison set forth above, or as defined as being capable of hybridizing to a target nucleic acid segment under relatively stringent conditions such as those described herein.

Alternatively, the hybridizing segments may be shorter oligonucleotides. Sequences of 17 bases long should occur only once in the human genome and, therefore, suffice to specify a unique target sequence. Although shorter oligomers are easier to make and increase *in vivo* accessibility, numerous other factors are involved in determining the specificity of hybridization. Both binding affinity and sequence specificity of an oligonucleotide to its complementary target increases with increasing length. It is contemplated that exemplary oligonucleotides of 8, 9, 10, 11, 12, 13, 14, 15, 16, 17, 18, 19, 20, 25, 30, 35, 40, 45, 50, 55, 60, 65, 70, 75, 80, 85, 90, 95, 100 or more base pairs will be used, although others are contemplated.

Suitable hybridization conditions will be well known to those of skill in the art. In certain applications, for example, substitution of amino acids by site-directed mutagenesis, it is appreciated that lower stringency conditions are required. Under these conditions, hybridization may occur even though the sequences of probe and target strand are not perfectly complementary, but are mismatched at one or more positions. Conditions may be rendered less stringent by increasing salt concentration and decreasing temperature. For example, a medium stringency condition could be provided by about 0.1 to 0.25 M NaCl at temperatures of about 37°C to about 55°C, while a low stringency condition could be provided by about 0.15 M to about 0.9 M salt, at temperatures ranging from about 20°C to about 55°C. Thus, hybridization conditions can be readily manipulated, and thus will generally be a method of choice depending on the desired results.

In other embodiments, hybridization may be achieved under conditions of, for example, 50 mM Tris-HCl (pH 8.3), 75 mM KCl, 3 mM MgCl₂, 10 mM dithiothreitol, at temperatures between approximately 20°C to about 37°C. Other hybridization conditions utilized could include approximately 10 mM Tris-HCl (pH 8.3), 50 mM KCl, 1.5 µM MgCl₂, at temperatures ranging from approximately 40°C to about 72°C. Formamide and SDS also may be used to alter the hybridization conditions.

Peptide nucleic acids (PNA) are DNA mimics with a pseudopeptide backbone which form stable duplex structures with complementary DNA, RNA (or PNA) oligomers. PNA's were originally used as ligands for the recognition of double-stranded DNA. The nucleobases of DNA were retained, but the deoxyribose phosphodiester backbone of DNA was replaced by a pseudo-peptide backbone that was homomorphous with the DNA backbone. The theory was that the neutral peptide backbone would improve triplex binding. However, PNA's could also mimic single stranded nucleic acids by default. They now are used as clamping agents in PCR reactions.

LNA's, or "Locked Nucleic Acids" are a nucleic acid analogs containing a 2'-O, 4'-C methylene bridge, which restricts the flexibility of the ribofuranose ring and locks the structure into a rigid bicyclic formation. This confers enhanced hybridization performance and exceptional biostability. They can be designed to enhance a wide variety of genomic applications and technologies that rely upon the use of oligonucleotides: general hybridization probes, probes for *in situ* hybridization, capture probes for target enrichment, allele-specific PCR™, antisense, and strand invasion techniques.

### III. Formulations and Routes for Administration to Patients

Where clinical applications are contemplated, it will be necessary to prepare pharmaceutical compositions - expression vectors, virus stocks, proteins, antibodies and drugs - in a form appropriate for the intended application. Generally, this will entail preparing compositions that are essentially free of pyrogens, as well as other impurities that could be harmful to humans or animals. The skilled artisan is directed to "Remington's Pharmaceutical Sciences" 15th Edition, incorporated herein by reference. Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

One will generally desire to employ appropriate salts and buffers to render delivery vectors stable and allow for uptake by target cells. Buffers also will be employed when recombinant cells are introduced into a patient. Aqueous compositions of the present invention comprise an effective amount of the vector to cells, dissolved or dispersed in a pharmaceutically acceptable carrier or aqueous medium. Such compositions also are referred to as inocula. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well know in the art. Except insofar as any conventional media or agent is incompatible with the vectors or cells of the present invention, its use in therapeutic compositions is contemplated. Supplementary active ingredients also can be incorporated into the compositions.

The active compositions of the present invention may include classic pharmaceutical preparations. Administration of these compositions according to the present invention will be via any common route so long as the target tissue is available via that route. This includes oral, nasal, buccal, rectal, vaginal or topical. Alternatively, administration may be by orthotopic, intradermal, subcutaneous, intramuscular, intraperitoneal or intravenous injection. Such compositions would normally be administered as pharmaceutically acceptable compositions, described *supra.* Of particular interest is direct intratumoral administration, perfusion of a tumor, or admininstration local or regional to a tumor, for example, in the local or regional vasculature or lymphatic system.

The active compounds may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For oral administration the polypeptides of the present invention may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient may be added in a therapeutically effective amount to a paste dentifrice that may include water, binders, abrasives, flavoring agents, foaming agents, and humectants.

The compositions of the present invention may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1: PATIENTS AND METHODS

**Patients with pancreatic cancer**. Twenty-three patients who underwent curative resection for PDAC from 1996-2004 were initially evaluated as a pilot cohort for this research study. Patient specimens were obtained from John Wayne Cancer Institute (JWCI) and UCLA School of Medicine (Los Angeles, CA). After completing analysis of the pilot cohort, 47 additional patients who underwent surgery for PDAC were accrued. Only patients with an adequate follow-up interval (*i.e*., ≥ 36 mos or until expiration) were selected. Patients were excluded if the final permanent section of either surgical margin (pancreatic transection or retroperitoneal) was histopathology positive by H&E, if either margin was unavailable for analysis, or if the patient died within 30 postoperative days. All patients, regardless of stage or nodal status, were offered adjuvant chemotherapy with combinations of 5-fluorouracil, leucovorin, mitomycin C, dipyridamole, and gemcitabine. Patients were offered adjuvant radiotherapy at the discretion of their individual physicians. A human subjects IRB approval was obtained at the respective institutions for the purposes of this study. Patient records, including radiographic images (when available), were reviewed. All patient demographics are listed in Table 1.

**Table 1- Patient Demographics**

| **Clinicopathologic Factors** | **Patients (n)** |
|---|---|
| | |
| **Total Patients** | **70** |
| Men | **35** |
| Women | **35** |
| **Age (yr)** | |
| Mean | **67** |
| Range | **42-90** |
| **Surgical Procedures** | |
| Pancreaticoduodenectomy | **68** |
| Distal Pancreatectomy | **2** |
| **AJCC stages** | |
| pl | **26** |
| pll | **44** |
| **Primary Tumor** | |
| T₁ | **16** |
| T₂ | **46** |
| T₃ | **8** |
| **Lymph Node Metastasis** | |
| N₀ | **30** |
| N₁ | **40** |
| **Pathologic Grade** | |
| Well | **11** |
| Moderate | **33** |
| Poor | **26** |
| **Tumor Size (cm)** | |
| 0-2 | **20** |
| >2-5 | **46** |
| >5 | **4** |
| **Perineural Invasion** | |
| Absent | **24** |
| Present | **46** |
| **Lymphovascular Invasion** | |
| Absent | **52** |
| Present | **18** |
| | |

**Pancreatic cancer specimens.** Paraffin-embedded archival tissues (PEAT) of primary and margin PDAC from the cohort of 70 patients were evaluated at JWCI. Prior to study inclusion, archived H&E margin slide sections were reviewed by microscopy to confirm the absence of cancer cells in the surgical margins. Immunohistochemistry was not utilized to re-evaluate any of these surgical margins. Sections (three 10-µm cuts) of PEAT primary tumor and margin were then obtained and stored in sterile microcentrifuge tubes (Eppendorf Biopur, Westbury, NY). After procurement of paraffin sections for PCR™ analysis, an additional paraffin section (5 µm) from the margin block was cut and stained by H&E and examined by a surgical pathologist to further confirm the absence of cancer cells. DNA was extracted using a modified assay (QIAamp DNA Mini Kit, Qiagen Inc., Valencia, CA) as previously described (Hoon *et al.,* 2004; Fujimoto *et al.,* 2004). When tumor was present in the margin blocks (*e.g*., radial retroperitoneal margins), PEAT sections (4x5 µm) were placed on microscope slides and histologically benign tissue was macrodissected or procured with the aid of laser capture microdissection (LCM; Arcturus, Mountain View, CA) as previously described (Hoon *et al.,* 2002; Nakayama *et al.,* 2001).

**KRAS mutation in pancreatic cancer cell lines.** Established pancreatic cancer cell lines (MIA PaCa2, PANC-1, Hs 766T, and BxPC-3) were obtained from the American Type Culture Collection (ATCC; Manassas, VA) and cultured as recommended by ATCC. MIA PaCa2 and PANC-1 have *KRAS* mutations (GGT→TGT and GGT→GAT, respectively) at codon 12, whereas Hs 766T and BxPC-3 have wild-type (wt) *KRAS.* These cell lines were used as positive and negative cancer controls. Mutations in codons 13 and 61 were not assessed because of the overwhelming predominance of codon 12 mutations in PDAC (Cubilla *et al.,* 1975; Hruban *et al.,* 2000; Hruban *et al.,* 2000; Kitago *et al.,* 2004).

An immortalized normal human pancreas ductal epithelial (HPDE) cell line was kindly provided by Dr. Tsao (University of Toronto, Toronto) and was used as a wtDNA control (Ouyang *et al.,* 2000; Furukawa *et al.,* 1996). All cells were incubated at 37°C with 5% CO₂. DNA from cell lines was extracted, isolated, and purified using DNAZol (Molecular Research Center Inc., Cincinnati, OH) as previously described (Spugnardi *et al.,* 2003). PEAT and cell line DNA was quantified and assessed for purity by ultraviolet spectrophotometry and PICOGreen detection assay (Molecular Probes, Eugene, OR) as previously described (Spugnardi *et al.,* 2003).

**Primers and Probes and PCR assay.** Detection of the *KRAS* mutation was performed using a peptide nucleic acid (PNA) PCR™ assay that was previously established to specifically detect *KRAS* mutations at codon 12 in PEAT primary colorectal cancers and lymph node metastases (Taback *et al.,* 2004). The PNA clamp, which has a higher binding affinity for DNA than PCR primers, was designed for complementary hybridization to the wt*KRAS* allele (Faruqi *et al.,* 1998). By hybridizing to the wtDNA template, it inhibits annealing of the partially overlapping reverse primer and thus inhibits the amplification of the wt*KRAS*. Because the PNA/DNA hybrid is unstable due to the base pair mismatch, it does not anneal to and inhibit the amplification of mutant *KRAS* (Faruqi *et al.,* 1998). The sensitivity of the PNA PCR™ assay to detect micrometastases with *KRAS* mutation has been previously demonstrated (Taback *et al.,* 2004). Results of the PNA PCR™ assay were analyzed and expressed by binary (+/-) values.

Real-time quantitative PCR™ (qRT) was performed using the following primers: *KRAS,* 5'- CGCTCACTGCGCTCAACAC - 3' (forward) (SEQ ID NO:1) and 5' - TCAGGCGGCCGCACACCT - 3' (reverse) (SEQ ID NO:2); FRET probe, 5'-FAM-CATTCTGTGCCGCTGAGCCG - BHQ -1-3' (SEQ ID NO:3); PNA, TACGCCACCAGCTCC (SEQ ID NO:4). The qRT assay was performed with the iCycler iQ RealTime PCR™ Detection System (Bio-Rad Laboratories, Hercules, CA). Genomic DNA (20 ng) was amplified by qRT in a 20 µL reaction containing 1µm of each primer, 1.75 µM PNA, 200 µM of each deoxynucleotide triphosphate, 4.0 mM MgCl_{2,} 10X AmpIiTaq Buffer, and 1 unit of *AmpliTaq* Gold Polymerase (Applied Biosystems, Branchburg, NJ). Each PCR™ reaction was subjected to 40 cycles at 94°C for 60 seconds, 70°C for 50 seconds, and 58°C for 50 seconds and 72°C for 60 seconds. PCR™ reactions were also performed without PNA to amplify wt*KRAS* and verify DNA integrity. Each sample was assayed in triplicate with positive and negative controls as previously described (Taback *et al.,* 2004).

***KRAS* sequencing.** Representative *KRAS*-positive and -negative specimens (n=16) were directly sequenced on both strands to confirm the accuracy of the PNA clamp method as previously described (Taback *et al.,* 2004). The following *KRAS* primers were used for PCR™ amplification: 5'-GGTACTGGTGGAGTATTTGATAGTG-3' (forward) (SEQ ID NO:5) and 5'- TGGATCATATTCGTCCACAAAA-3' (reverse) (SEQ ID NO:6). Each PCR™ reaction mixture was initially heated to 94°C for 10 minutes and was then subjected to 32-40 cycles at 94°C for 30 seconds, 64°C for 30 seconds, and 72°C for 7 minutes. PCR™ products were purified with QIAquick PCR™ Product Purification Kit (Qiagen), and then direct-sequenced using DTCS Quick Start kit (Beckman Coulter; Fullerton, CA) with an annealing temperature of 58°C. Dye-terminated products were precipitated by ethanol and separated by capillary electrophoresis on a CEQ8000XL automated sequencer (Beckman Coulter).

**Statistical analysis.** Patient characteristics and detection of *KRAS* mutation were summarized using mean, median, and frequency. Clinicopathologic factors of patients with positive or negative *KRAS* mutation were compared by Fisher's Exact test and Student's T-test. Survival curves with respect to *KRAS* mutation were constructed using Kaplan-Meier's method. The log-rank test was used to compare the equality of the two curves. Univariate analysis of prognostic factors included age, gender, stage, tumor extent, lymph node status, grade, tumor size, perineural invasion, and lymphovascular invasion. The Cox proportional hazard regression model was used to evaluate the prognostic significance of *KRAS* mutation when clinical prognostic factors were adjusted. A stepwise method was chosen for covariate selection. All analyses were performed using SAS (SAS /STAT User's Guide, version 8; SAS Institute Inc, Cary, NC, USA) and tests were 2-sided and were considered significant when P values were ≤ 0.05.

### EXAMPLE 2: RESULTS

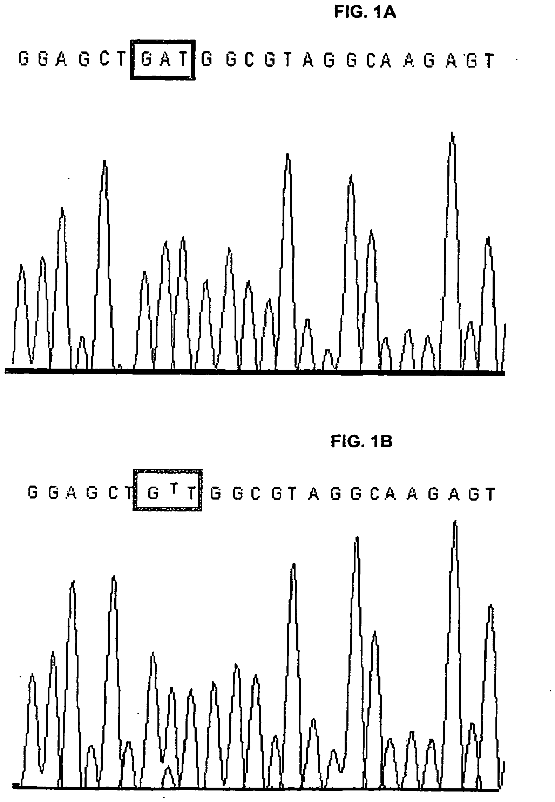

**PNA PCR accuracy.** The accuracy of the PNA clamp method was previously assessed in *KRAS*-positive (n=16) and *KRAS*-negative (n=17) PEAT colorectal cancer specimens (Balcom *et al.,* 2001). The sensitivity of the assay was determined by detection of KRAS mutation in micrometastatic (Greene *et al.,* 2002) colorectal cancer lymph node lesions. For all 33 cancer specimens, direct sequencing validated the results of the PNA PCR™ assay, illustrating 100% specificity and sensitivity (Taback *et al.,* 2004). This previous study utilized PCR™ amplification of *KRAS* mutation followed by a semiquantitative, electrochemiluminescent assay to detection the KRAS mutation. The current study was adapted to utilize the same *KRAS* PCR™ primers, PNA, and amplification conditions on a more efficient, sensitive, and high-throughput, real-time quantitative PCR™ platform. To validate the accuracy of the real-time PCR PNA clamp assay, the inventors analyzed 16 representative *KRAS*-positive (n=8) and *KRAS*-negative (n=8) PEAT PDAC specimens. The wild-type *KRAS* DNA sequence at codon 12 is guanine-guanine-thymine (GGT). Five different *KRAS* mutations at codon 12 were detected (GAT, n=2; GGG, n=2; GTT, n=2; TGT, n=1; and TGG, n=1) from eight *KRAS*-positive specimens. Representative sequences of *KRAS* mutations are presented in FIGS. 1A-B.

**KRAS mutation in patients with pancreatic cancer.** Twenty-three patients with PDAC were initially analyzed as the pilot cohort. *KRAS* mutations were detected in 83% (n=19 of 23) of the primary tumors and 48% (n=11 of 23) of either surgical margin. The pancreatic transection and retroperitoneal margins were *KRAS*-positive in four and eight patients, respectively; both margins were *KRAS*-positive in one patient. After analysis of this pilot cohort, an additional 47 patients were accrued. For all 70 patients, the median survival was 21 months at a median follow-up period of 17 months. The 5-yr overall survival rate was 19%. Patients were treated with pancreaticoduodenectomy (n=68) or distal pancreatectomy (n=2); none underwent total resection of the pancreas. One patient had segmental resection of the superior mesenteric-portal vein confluence. At the time of analysis, 45 (64%) of the 70 patients had succumbed to disease.

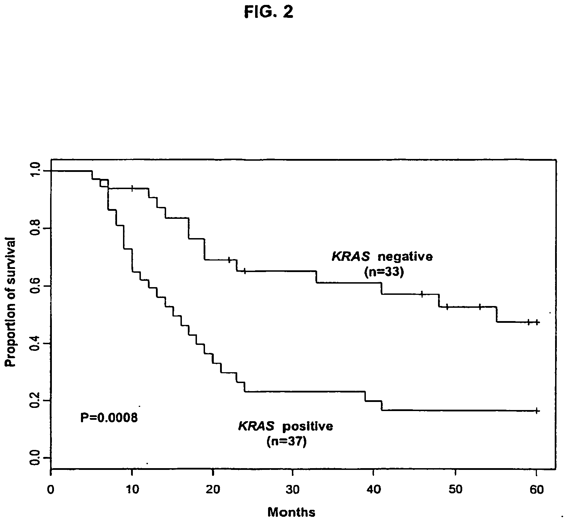

Overall, 57 (81%) of the 70 patients had the *KRAS* mutation in the primary tumor. This percentage is consistent with previous reports of an incidence from 75% to 100% (Almoguera *et al.,* 1888; Smit *et al.,* 1988; Kitage *et al.,* 2004; Hruban *et al.,* 2001; Wanebo and Vezeridis, 1996; Longnecker and Terhune, 1998; Mu *et al.,* 2004). *KRAS* mutation was detected in either margin in 37 (53%) patients. *KRAS*-positive margins were detected in pancreatic transections (n=17), retroperitoneal margins (n=27), or in both margins (n=7). Generally, most surgical margins had evidence of low-grade PanIN (Hruban *et al.,* 2004); high grade PanINs were rare. When patients were categorized into two groups based on *KRAS* margin status, there was a higher incidence of perineural invasion and a lower rate of lymphovascular invasion in the *KRAS*-positive group (Table 2). Kaplan-Meier curves showed significant difference in overall survival for patients with *KRAS* mutation-positive vs - negative margins (median, 15 vs 55 months, respectively; log-rank, p=0.0008; FIG. 2).

**Table 2 - Comparison of Clinicopathologic Factors for KRAS Positive and Negative Patients**

| **Clinicopathologic Factors** | ***KRAS*** | | ***P-value** |
|---|---|---|---|
| | **Negative** (n=33) | **Positive** (n=37) | |
| **Age (yr)** | | | 0.075 |
| Mean ± SD | 65±9 | 69±11 | |
| Range | 45-78 | 42-90 | |
| **Gender** | | | 1.0 |
| Female | 17 | 18 | |
| Male | 16 | 19 | |
| **AJCC Stage** | | | 0.33 |
| pl | 10 | 16 | |
| pll | 23 | 21 | |
| **Primary Tumor** | | | 0.074 |
| T₁ | 11 | 5 | |
| T₂ | 19 | 27 | |
| T₃ | 3 | 5 | |
| **Lymph Node Metastasis** | | | 0.15 |
| N₀ | 11 | 19 | |
| N₁ | 22 | 18 | |
| **Tumor Size (cm)** | | | 0.16 |
| 0-2 | 12 | 8 | |
| >2-5 | 20 | 26 | |
| >5 | 1 | 3 | |
| **Pathologic Grade** | | | 0.56 |
| well | 3 | 8 | |
| moderate | 21 | 12 | |
| poor | 9 | 17 | |
| **Perineural Invasion** | | | 0.024 |
| absent | 16 | 8 | |
| present | 17 | 29 | |
| **Lymphovascular Invasion** | | | 0.027 |
| absent | 29 | 23 | |
| present | 4 | 14 | |

| | | | |
|---|---|---|---|
| *Comparison for age was performed by Student's t-test; the remaining clinical factors were compared by Fisher's Exact test. | | | |

By univariate analysis, *KRAS* mutation, grade, and perineural invasion were significant factors for disease outcome (Table 3). When clinicopathologic factors were adjusted, multivariate analysis identified *KRAS* mutation as a significant prognostic factor for poor survival (HR 2.7, 95% CI: 1.4-5.5; p=0.004) (Table 3). Tumor grade and perineural invasion were also significant factors for poor survival (HR 6.7, 95% CI: 2.4-18.5, p=0.0001; and HR 2.1, 95% CI: 1.0-4.2, p=0.04, respectively).

**Table 3 - Univariate and Multivariate Analyses**

| | **Univariate Analysis** | | | **Multivariate Analysis** | |
|---|---|---|---|---|---|
| | **Death/ N** | **Hazard ratio (95% Cl)** | **P-value** | **Hazard ratio (95% Cl)** | **P-value** |
| **Age (yrs)** | | | NS | 1.0 | NS |
| <50 | 3/4 | 1.0 | | | |
| 50-70 | 24/37 | 1.2 (0.4-3.6) | | | |
| >70 | 18/29 | 1.5 (0.4-5.2) | | | |
| **Sex** | | | NS | 1.0 | NS |
| Female | 22/35 | 1.0 | | | |
| Male | 23/35 | 1.2 (0.6-2.1) | | | |
| **Stage** | | | NS | 1.0 | NS |
| p1 | 17/26 | 1.0 | | | |
| p2 | 28/44 | 1.2 (0.6-2.2) | | | |
| **Tumor extent** | | | NS | 1.0 | NS |
| T₁ | 9/16 | 1.0 | | | |
| T₂ | 32/46 | 1.5 (0.7-3.2) | | | |
| T₃ | 4/8 | 0.8 (0.2-2.6) | | | |
| **Lymph Node** | | | NS | 1.0 | NS |
| **Disease** | 18/30 | 1.0 | | | |
| T₀ | 27/40 | 1.4 (0.8-2.6) | | | |
| T₁ | | | | | |
| **Tumor Size (cm)** | | | NS | 1.0 | NS |
| 0-2 | 12/20 | 1.0 | | | |
| >2-5 | 29/46 | 1.2 (0.6-2.4) | | | |
| >5 | 4/4 | 3.3 (1.0-10.4) | | | |
| **Tumor Grade** | | | | | |
| well | 6/11 | 1.0 | | 1.0 | |
| moderate | 16/33 | 1.4 (0.5-3.5) | | 2.6 (0.9-7.3) | |
| poor | 23/26 | 4.9 (1.9-12.9) | 0.0001 | 6.7 (2.4-18.5) | 0.0001 |
| **Perineural Invasion** | 13/24 | 1.00 | | 1.0 | |
| No | 32/46 | 2.1 (1.1-4.2) | 0.02 | 2.1 (1.0-4.2) | 0.04 |
| Yes | | | | | |
| **Lymphovascular Invasion** | | | NS | 1.0 | NS |
| No | 33/52 | 1.0 | | | |
| Yes | 12/18 | 1.5 (0.8-2.9) | | | |
| ***KRAS* in margins** | | | | | |
| No | 17/33 | 1.00 | | 1.0 | |
| Yes | 28/37 | 2.8 (1.5-5.2) | 0.0009 | 2.7 (1.4-5.5) | 0.004 |

### EXAMPLE 3: DISCUSSION

The value of obtaining histopathologically negative surgical margins has been borne out from subset analyses of large cohort reviews and randomized controlled trials evaluating treatment for PDAC (Yeo *et al.,* 1997; Neoptolemos *et al.,* 2001; Balcom *et al.,* 2001). The inventors used PCR™ to detect *KRAS* mutation in surgical margins of over half the patients (53%) in this study cohort, all of whom had negative H&E histopathology. Correlation of *KRAS* margin status with clinical outcome demonstrated significant difference in overall survival. These findings suggest that intraoperative determination of surgical margin adequacy is not sensitive to identify relevant genetic aberrancies of PDAC that may be present as field defects or occult neoplastic cells.

A high rate of locoregional recurrence and concomitant short survival support the existence of field cancerization or occult spread of neoplastic cells in PDAC. In order to correlate PCR™ detection of *KRAS* mutation with clinical data regarding cancer recurrences, the inventors reviewed radiographic reports and interventional studies for patients with *KRAS-*positive margins and poor survival. Because most patients were referred from outside institutions, radiographic data was usually unavailable and the inventors could not determine disease-free interval for the entire cohort. However, in nine patients with *KRAS* positive margins and poor survival, radiographic imaging studies and peritoneal cytology revealed local recurrence (n=6) or malignant ascites (n=3), respectively. This may suggest that surgical resection tailed to remove all cells with genetic aberrancies. In studies of patients with head and neck cancers (Tabor *et al.,* 2004; Brennan *et al.,* 1995; Goldenberg *et al.,* 2004; Koch *et al.,* 1994) have provided evidence to support this suggestion. They found that patients whose surgical margins harbored LOH or *p53* DNA mutation had recurrence at those margins and had worse disease outcomes than patients without such genetic aberrancies at the margins.

*KRAS* DNA mutation was chosen for this study because of its frequent occurrence in pancreatic cancers and its potential pathogenetic role (Hingorani *et al.,* 2003; Almoguera *et al.,* 1988; Smit *et al.,* 1988; Hruban *et al.,* 2001; Wanebo and Vezeridis, 1996; Longnecker and Terhune, 1998; Mu *et al.,* 2004). Although recent evidence demonstrates that *KRAS* DNA mutation may be an essential precursor of pancreatic malignancy Hingorani *et al.,* 2003), there are reports of *KRAS* mutations in benign pancreatic disorders (Luttges *et al.,* 1999; Rivera *et al.,* 1997; Tada *et al.,* 1996). Moreover, *KRAS* mutations have been detected in PanIN lesions (Hruban *et al.,* 2001; Hruban *et al.,* 2004) and were generally present in all the margin specimens of this study cohort. The relationship between PanIN, *KRAS* mutation, and surgical margins remains unclear. A recent report on colorectal cancer by Yamada *et al.* (2005) contends that *KRAS* mutation is a field cancerization change in normal tissues surrounding primary tumors.

The inventors are currently investigating whether additional genetic or epigenetic aberrancies are present in PDAC margin tissues. They have relied on PCR™ assays to detect such defects, because sensitive and efficient diagnostic antibodies are unavailable at this time. Utilizing PCR techniques, the inventors have detected *KRAS* mutation in the surgical margins and hypothesize that the cells with *KRAS* may harbor additional discrete genetic abnormalities. *KRAS*-positive margins in patients with PDAC could indicate tumors that have intrinsically worse biology of disease. As such, the detection of *KRAS* mutations in the surgical margins could be reflective of more aggressive tumors, the end-product of which may be either field cancerization or occult spread of tumor cells. Furthermore, tumors with *KRAS-*positive margins may potentially represent PDAC with occult spread of neoplastic cells throughout the entire pancreas or to distant organs. The prognostic significance of grade and perineural invasion by multivariate analysis supports the contention of worse tumor biology (Table 3). To the contrary, five patients with *KRAS*-positive margins with survival exceeding 5 years were noted in this study cohort. Whether field cancerization or occult spread of neoplastic cells extends beyond the standard margins of resection and whether wider, PCR-negative surgical margins could affect PDAC outcome will require evaluation in a prospective study.

Though field cancerization or occult spread of neoplastic cells may occur in many cancers, (Braakhuis *et al.,* 2003) the anatomic limitations of the retroperitoneal pancreas make it particularly problematic in patients with PDAC. Of note, 27 (73%) of the 37 positive margins were from the retroperitoneum. Even when total pancreatectomy has been performed for PDAC, the survival data are no better (Karpoff *et al.,* 2001), an outcome which, perhaps, can be explained by the high retroperitoneal *KRAS*-positive rate. Radiation therapy may appear attractive when surgical margins are positive; however, treatment outcomes have been mixed and a recent large, randomized controlled trial has demonstrated no survival advantage in patients receiving radiation therapy (Neoptolemos *et al*., 2004).

The development of an expanding field of genotypically altered-benign or malignant cells has important clinical consequences. The inventors demonstrate in patients with PDAC that field cancerization or occult spread of neoplastic cells may extend to the margins of surgical resection and correlates strongly with clinical outcomes. A recent study by Pantel *et al.* (2004) demonstrates the importance of molecular techniques in identifying occult spread of tumor cells in PDAC. Surgical treatment of PDAC, therefore, may require particular scrutiny of the margins of resection beyond the use of standard histopathologic techniques. The current study was performed using PEAT PDAC specimens.

### XI. References

The following references, to the extent that they provide exemplary procedural or other details supplementary to those set forth herein, are specifically incorporated herein by reference.
U.S. Patent 4,683,195
U.S. Patent 4,683,202
U.S. Patent 4,800,159
U.S. Patent 4,883,750
U.S. Patent 5,279,721
Abbruzzese et al., Anticancer Res., 17:795-801, 1997.
Almoguera et al., Cell, 53:549-554, 1988.
Balcom et al., Arch. Surg., 136:391-398, 2001.
Bellus, J. Macromol. Sci. Pure Appl. Chem., A31(1): 1355-1376, 1994.
Bogoevski et al., Ann. Surg., 240:993-1000, 2004.
Braakhuis et al., Cancer Res., 63:1727-1730, 2003.
Brennan et al., N. Engl. J. Med., 332:429-435, 1995.
Caldas et al., Cancer Res., 54:3568-3573, 1994.
Califano et al., Cancer Res., 56:2488-2492, 1996.
Cubilla et al., Cancer Res., 35:2234-2248, 1975
Culver et al., Science, 256(5063):1550-1552, 1992.
Deng et al., Science, 274:2057-2059, 1996.
European Appln. 320 308
European Appln. 329 822
Evans et al., In: Current Surgical Therapy, Cameron (Ed.), MO, Mosby, 558-567, 2001.
Faruqi et al., Proc. Natl. Acad. Sci. USA, 95:1398-1403, 1998.
Feinberg & Vogelstein, Anal. Biochem., 132(1):6-13, 1983.
Fodor et al., Biochemistry, 30(33):8102-8108, 1991.
Freifelder, In: Physical Biochemistry Applications to Biochemistry and Molecular Biology, 2nd Ed. Wm. Freeman and Co., NY, 1982.
Frohman, In: PCR Protocols: A Guide To Methods And Applications, Academic Press, N.Y., 1990.
Fujimoto et al., Cancer Res., 64:2245-2250, 2004.
Furukawa et al., Am. J. Pathol., 148:1763-1770, 1996.
Garcia et al., J. Pathol., 187:61-81, 1999.
GB Appln. 2 202 328
Goldenberg et al., Arch. Otolaryngol Head Neck Surg., 130:39-44, 2004.
Greene et al., In: AJCC Cancer Staging Manual, 6th Ed., NY, Springer, 179-188, 2002.
Griffin et al., Cancer, 66:56-61, 1990.
Guo et al., Clin. Cancer Res., 10:5131-5136, 2004.
Hacia et al., Nature Genet., 14:441-449, 1996.
Hanahan and Weinberg, Cell, 100:57-70, 2000.
Harlow and Lane, In: Antibodies: A Laboratory Manual, Cold Spring Harbor Laboratory, Cold Spring Harbor, NY, 346-348, 1988.
Hingorani et al., Cancer Cell. 4:437-450, 2003.
Hirai et al., Biochem. Biophys. Res. Commun., 127:168-174, 1985.
Hoon et al., Methods Enzymol., 356:302-309, 2002.
Hoon et al., Oncogene, 23:4014-4022, 2004.
Hruban et al., Am. J. Pathol., 156:1821-1825, 2000a.
Hruban et al., Am. J. Surg. Pathol., 28:977-987, 2004.
Hruban et al., Cancers J., 7:251-258, 2001.
Hruban et al., Clin. Cancer Res., 6:2969-2972, 2000b.
Innis, et al., In: PCR Protocols. A guide to Methods and Application, Academic Press, Inc. San Diego, 1990.
Ito et al., Int. J. Cancer, 113:22-28, 2005.
Izawa et al., Cancer, 92:1807-1817, 2001.
Jassem et al., Cancer, 100:1951-1960, 2004.
Jemal et al., Cancer J. Clin., 55:10-30, 2005.
Karpoff et al., Arch. Surg., 136:44-47, 2001.
Kitago et al., Int. J. Cancer, 110:177-182, 2004.
Koch et al., Arch. Otolaryngol Head Neck Surg., 120:943-947, 1994.
Kwoh et al., Proc. Natl. Acad. Sci. USA, 86:1173, 1989.
Longnecker and Terhune, Pancreas, 17:323-324, 1998.
Luttges et al., Cancer, 85:1703-1710, 1999.
Masasyesva et al., Int. J. Cancer, 113:1022-1025, 2005.
Moinfar et al., Cancer Res., 60:2562-2566, 2000.
Mu et al., World J. Gastroenterol., 10:471-475, 2004.
Nakayama et al., Am. J. Pathol., 158:1371-1378, 2001.
Nathan et al., J. Clin. Oncol., 17:2909-2914, 1999.
Nathan et al., Laryngoscope, 112:2129-2140, 2002.
Nathan et al., Oncogene, 15:579-584, 1997.
Neoptolemos et al., Ann. Surg., 234:758-768, 2001.
Neoptolemos et al., N. Engl. J. Med., 350:1200-1210, 2004.
Nitecki et al., Ann. Surg., 221:59-66, 1995.
Ohara et al., Proc. Natl. Acad. Sci. USA, 86:5673-5677, 1989.
Ouyang et al., Am. J. Pathol., 157:1623-1631, 2000.
PCT Appln. WO 88/10315
PCT Appln. WO 89/06700
PCT Appln. WO 90/07641
Pease et al., Proc. Natl. Acad. Sci. USA, 91:5022-5026, 1994.
Pignon et al., Hum. Mutat., 3:126-132, 1994.
Remington's Pharmaceutical Sciences, 15th ed., 33:624-652, Mack Publishing Company, Easton, PA, 1980.
Remington's Pharmaceutical Sciences, 15th ed., pages 1035-1038 and 1570-1580, Mack Publishing Company, Easton, PA, 1980.
Rivera et al., Surgery, 121:42-49, 1997.
Sambrook et al., In: Molecular cloning: a laboratory manual, 2nd Ed., Cold Spring Harbor Laboratory Press, Cold Spring Harbor, NY, 1989.
Shoemaker et al., Nature Genetic, 14:450-456, 1996.
Slaughter et al., Cancer, 6:963-968, 1953.
Smit et al., Nucleic Acids Res., 16:7773-7782, 1988.
Sorenson, Clin. Cancer Res., 6:2129-2137, 2000.
Spugnardi et al., Cancer Res., 63:1639-1643, 2003.
Taback et al., Int. J. Cancer, 111:409-414, 2004.
Tabor et al., Clin. Cancer Res., 10:3607-3613, 2004.
Tada et al., Gastroenterology, 100:233-238, 1991.
Tada et al., Gastroenterology, 110:227-231,1996.
Temam et al., Clin. Cancer Res., 10:4022-4028, 2004.
Tian et al., Int. J. Cancer, 78:568-575, 1998.
Walker et al., Nucleic Acids Res., 20(7):1691-1696, 1992.
Wanebo and Vezeridis, Cancer, 78(3 Suppl):580-591, 1996.
Warshaw and Fernandez-del Castillo, N. Engl. J. Med., 326:455-465, 1992.
Wernert et al., Anticancer Res., 21:2259-2264, 2001.
Westerdahl et al., Hepatogastroenterology, 40:383-387, 1993.
Willett et al., Ann. Surg., 217:144-148, 1993.
Wu et al., Anal. Chem., 70:456A, 1998.
Yamada et al., Int. J. Cancer, 113:1015-1021, 2005,
Yeo et al., Ann. Surg., 226:248-257, 1997.

## Claims

1. A method of predicting (i) survival, (ii) cancer progression, and/or (iii) recurrence of cancer in a pancreatic cancer patient having undergone surgical resection, the method comprising the steps of:
(a) assessing a nucleic acid or protein in cells of a tissue sample obtained from the surgical margin with negative histopathology of H & E of a resected pancreatic tumor for the occurrence of a mutation in K-ras, wherein the occurrence of the mutation in the tissue sample is predictive of (i) decreased survival, (ii) cancer progression and/or (iii) recurrence of cancer; and
(b) predicting (i) survival, (ii) cancer progression, and/or (iii) recurrence of cancer in the cancer patient.

2. The method of claim 1, wherein step (a) of assessing comprises immunologic detection of a protein or detection of a nucleic acid.

3. The method of claim 2, wherein the nucleic acid is an RNA.

4. The method of claim 2, wherein the nucleic acid is a DNA.

5. The method of claim 2, wherein detection of the nucleic acid comprises nucleic acid amplification.

6. The method of claim 2, wherein detection of the nucleic acid comprises sequencing.

7. The method of claim 2, wherein detection of the nucleic acid comprises primer extension.

8. The method of claim 2, wherein detection of the nucleic acid comprises Northern or Southern blotting.

9. The method of claim 2, wherein detection of the nucleic acid comprises restriction endonuclease treatment.

## Patentansprüche

1. Ein Verfahren zur Vorhersage von (i) Überleben, (ii) Fortschreiten des Krebses und/oder (iii) Wiederauftreten des Krebses in einem Patienten mit Pankreaskrebs, der sich einer chirurgischen Resektion unterzogen hat, wobei das Verfahren die Schritte enthält:
(a) Beurteilen einer Nukleinsäure oder eines Proteins in einer Zelle einer Gewebeprobe erhalten aus dem Resektionsrand mit negativer Histopathologie durch H&E eines resezierten Pankreastumors über das Auftreten einer Mutation in K-ras, wobei das Auftreten der Mutation in der Gewebeprobe vorhersagend ist für (i) verringertes Überleben, (ii) Fortschreiten des Krebses und/oder (iii) Wiederauftreten des Krebses; und
(b) Vorhersagen von (i) Überleben, (ii) Fortschreiten des Krebses und/oder (iii) Wiederauftreten des Krebses in dem Krebspatienten.

2. Das Verfahren nach Anspruch 1, wobei der Schritt (a) des Beurteilens einen immunologischen Nachweis eines Proteins oder Nachweis einer Nukleinsäure enthält.

3. Das Verfahren nach Anspruch 2, wobei die Nukleinsäure eine RNA ist.

4. Das Verfahren nach Anspruch 2, wobei die Nukleinsäure eine DNA ist.

5. Das Verfahren nach Anspruch 2, wobei der Nachweis der Nukleinsäure Nukleinsäure-Amplifikation enthält.

6. Das Verfahren nach Anspruch 2, wobei der Nachweis der Nukleinsäure Sequenzieren enthält.

7. Das Verfahren nach Anspruch 2, wobei der Nachweis der Nukleinsäure Primer Extension enthält.

8. Das Verfahren nach Anspruch 2, wobei der Nachweis der Nukleinsäure Northern oder Southern Blotting enthält.

9. Das Verfahren nach Anspruch 2, wobei der Nachweis der Nukleinsäure Behandlung mit Restriktionsendonuklease enthält.

## Revendications

1. Méthode de prédiction (i) de la survie, (ii) de la progression du cancer, et/ou (iii) de la récurrence du cancer chez un patient atteint d'un cancer pancréatique ayant subi une résection chirurgicale, la méthode comprenant les étapes :
a) d'évaluation d'un acide nucléique ou d'une protéine dans les cellules d'un échantillon de tissu obtenu à partir du contour chirurgical ayant une histopathologie négative par H&E d'une tumeur pancréatique réséquée pour l'occurrence d'une mutation dans K-ras, où l'occurrence de la mutation dans l'échantillon de tissu est prédictif (i) d'une diminution de la survie, (ii) d'une progression du cancer et/ou (iii) d'une récurrence du cancer ; et
b) de prédiction (i) de la survie, (ii) de la progression du cancer, et/ou (iii) de la récurrence du cancer chez le patient atteint du cancer.

2. Méthode selon la revendication 1, dans laquelle l'étape (a) d'évaluation comprend la détection immunologique d'une protéine ou la détection d'un acide nucléique.

3. Méthode selon la revendication 2, dans laquelle l'acide nucléique est un ARN.

4. Méthode selon la revendication 2, dans laquelle l'acide nucléique est un ADN.

5. Méthode selon la revendication 2, dans laquelle la détection de l'acide nucléique comprend une amplification de l'acide nucléique.

6. Méthode selon la revendication 2, dans laquelle la détection de l'acide nucléique comprend un séquençage.

7. Méthode selon la revendication 2, dans laquelle la détection de l'acide nucléique comprend une extension des amorces.

8. Méthode selon la revendication 2, dans laquelle la détection de l'acide nucléique comprend un Northern ou un Southern Blot.

9. Méthode selon la revendication 2, dans laquelle la détection de l'acide nucléique comprend un traitement par une endonucléase de restriction.


### SEQUENCE LISTING

<110> HOON, DAVID S.B. KIM, JOSEPH
<120> MOLECULAR/GENETIC ABERRATIONS IN SURGICAL MARGINS OF RESECTED PANCREATIC CANCER REPRESENTS NEOPLASTIC DISEASE THAT CORRELATES WITH DISEASE OUTCOME
<130> JWCI:035WO
<140> UNKNOWN
   <141> 2006-06-27
<150> 60/694,383
   <151> 2005-06-27
<160> 6
<170> PatentIn Ver. 2.1
<210> 1
   <211> 19
   <212> DNA
   <213> Artificial Sequence
<220>
   <223> Description of Artificial Sequence: Synthetic Primer
<400> 1
   cgctcactgc gctcaacac 19
<210> 2
   <211> 18
   <212> DNA
   <213> Artificial Sequence
<220>
   <223> Description of Artificial Sequence: Synthetic Primer
<400> 2
   tcaggcggcc gcacacct 18
<210> 3
   <211> 20
   <212> DNA
   <213> Artificial Sequence
<220>
   <223> Description of Artificial Sequence: Synthetic Primer
<400> 3
   cattctgtgc cgctgagccg 20
<210> 4
   <211> 15
   <212> DNA
   <213> Artificial Sequence
<220>
   <223> Description of Artificial Sequence: Synthetic Primer
<400> 4
   tacgccacca gctcc 15
<210> 5
   <211> 25
   <212> DNA
   <213> Artificial Sequence
<220>
   <223> Description of Artificial Sequence: Synthetic Primer
<400> 5
   ggtactggtg gagtatttga tagtg 25
<210> 6
   <211> 22
   <212> DNA
   <213> Artificial Sequence
<220>
   <223> Description of Artificial Sequence: Synthetic Primer
<400> 6
   tggatcatat tcgtccacaa aa 22